# EUROPEAN PATENT APPLICATION

(11) **EP 3 520 930 A1**
(43) Date of publication of application: **07.08.2019**
(21) Application number: 19154595.3
(22) Date of filing: 30.01.2019
(51) Int. Cl.: B22F 3/105, B33Y 10/00, B33Y 30/00, B29C 64/153, B29C 64/357

(54) **PROCESS EQUIVALENT POWDER REUSE CAPSULE FOR ADDITIVE MANUFACTURING**

(30) Priority: 02.02.2018 US 201815886936
(71) Applicant: United Technologies Corporation, Farmington, CT 06032 (US)
(72) Inventor: BENNETT, Michael W, Lebanon, CT Connecticut 06249 (US); REBBECCHI, Thomas A, Hartford, CT Connecticut 06105 (US); O'NEILL, Christopher F, Hebron, CT Connecticut 06248 (US); BOYER, Jesse R, Middletown, CT Connecticut 06457 (US)
(74) Representative: Dehns

(57) **Abstract**

A system includes a build plate, at least one component that is additively manufactured on the build plate from a powder material, and at least one capsule that is additively manufactured on the build plate from the same powder material. The capsule includes an additively manufactured core that is representative of one or more scaled build configuration layers of the at least one component and an arrangement of the layers on the build plate, a sample of unused powder material, and a shell that at least partially encloses the core and the sample.

## Description

### BACKGROUND

Additive manufacturing techniques and systems are used to manufacture/fabricate various components, such as for example components of an engine. U.S. patent application publication number 2016/0326880 describes and illustrates various additive manufacturing systems and techniques.

A powder-based additive manufacturing technique is capable of manufacturing a number of engine components/assemblies. The technique is operative on the basis of a repeated application of metal powder layers and laser or electron-beam fusion of specified areas of a workpiece. Of the powder that is deposited into/onto the build chamber/table/plate, a portion of the powder is fused to form the component and a remainder of the powder is designated as used/residual.

Due to the high cost of new powder (sometimes referred to as "virgin powder") and variation of the powder characteristics from lot-to-lot, it is often desirable to reuse/recycle the residual powder in order to reduce cost. Blending is a technique of adding and homogenizing new/virgin powder with used/residual powder. However, the potential reuse of powder imposes its own set of challenges that need to be addressed. For example, used/residual powder characteristics are impacted by an amount of peripheral exposure (e.g., laser or electron beam exposure) during fusion of nearby powder, powder handling techniques, and a machine's processing environment. Therefore, the residual powder characteristics are unique to every build configuration. As used herein, a build configuration refers to the material (e.g., powder) that is deposited onto the build plate, an identification of the (type of) component(s) that are being built on the build plate, the quantity of said components being built, and an orientation associated with the component(s). Component build quality may deviate from a threshold (e.g., may be outside of an allowable tolerance) if the powder characteristics are not controlled/regulated.

With powder characterization methods and modeling tools becoming increasingly available, an opportunity exists for the definition of more effective and efficient additive manufacturing powder reuse standards for the production of high-quality components based on residual powder characteristics. Current guidelines for powder reuse focus on the number of times a powder can be reused before appreciable powder degradation; this is herein referred to as a reuse limit or reuse number. These techniques for the establishment of numerical reuse limits based on part quality allowances involve extensive testing of a non-ç-(é"'(tyyyhn,,,:ll) standard and often non-representative sample of the residual powder for a given build configuration (before and after blend with additional virgin powder). A numerical limit is established which then defines how many times residual powder can be reused before it needs to be discarded or reallocated to an alternative use/purpose (e.g., a manufacture of a different type of component). Establishment of a numerical reuse limit for a specified build geometry happens despite the fact that every build configuration has a different fusion source path (e.g., a different laser or electron beam path) and therefore a different impact on the bystander residual powder on the build plate. The result is that a large amount of powder and material specimen testing is conducted to establish numerical powder reuse limitations for a specific part geometry that cannot reliably be applied to any other build/production run. This "point-solution" methodology inhibits effective and efficient powder reuse because a reuse qualification characterization must be repeated for each unique build configuration. A more robust statistical approach to powder sampling and analysis is proposed to permit generalization of powder reuse limitations to various build configurations of the same geometry.

### BRIEF SUMMARY

The following presents a simplified summary in order to provide a basic understanding of some aspects of the disclosure. The summary is not an extensive overview of the disclosure. It is neither intended to identify key or critical elements of the disclosure nor to delineate the scope of the disclosure. The following summary merely presents some concepts of the disclosure in a simplified form as a prelude to the description below.

Aspects of the disclosure are directed to a system comprising: a build plate, at least one component that is additively manufactured on the build plate from a powder material, and at least one capsule that is additively manufactured on the build plate from the same powder material that includes an additively manufactured core that is representative of one or more scaled build configuration layers of the at least one component and an arrangement of the layers on the build plate, a sample of unused powder material, and a shell that at least partially encloses the core and the sample. In some embodiments, the shell completely encloses the core and the sample. In some embodiments, the shell includes a perforation to enable access to the sample. In some embodiments, the system comprises a cover that when coupled to the shell completely encloses the core and the sample, and the cover is removable from the shell to enable access to the sample. In some embodiments, the at least one component includes a plurality of components. In some embodiments, the system comprises a power source that is applied to the powder material, wherein a geometry of the core is based on a scaling factor derived from at least one of: a density of the powder material, a density of the plurality of components relative to a surface area of the build plate, an orientation of the plurality of components, a type of the components, and a spacing between the plurality of components. In some embodiments, each of the plurality of components is an instance of a common type of component. In some embodiments, components that are adjacent to one another on the build plate are equidistantly spaced from one another. In some embodiments, the capsule is shaped as at least one of a cylinder or a cube.

Aspects of the disclosure are directed to a capsule comprising: a core that is representative of one or more scaled build configuration layers of at least one additively manufactured component, a sample of unused powder material captured in-situ during fabrication of the at least one additively manufactured component, and a shell that at least partially encloses the core and the sample, wherein the sample has properties that are statistically representative of an entire population of the unused powder material on a powder bed. In some embodiments, the capsule is shaped as at least one of a cylinder or a cube. In some embodiments, the shell completely encloses the core and the sample. In some embodiments, the core is representative of a plurality of scaled build configuration layers of a plurality of additively manufactured components, the plurality of additively manufactured components including the at least one additively manufactured component. In some embodiments, the shell is made of at least a portion of the powder material.

Aspects of the disclosure are directed to a method comprising: fabricating at least one component from a powder material as a plurality of layers via a powder bed fusion additive manufacturing technique, and generating a capsule concurrent with the manufacture of the at least one component, the capsule including a core that is representative of one or more scaled build configuration layers of the at least one component, a sample of the powder material, and a shell that at least partially encloses the sample. In some embodiments, the shell completely encloses the core and the sample, and the method comprises opening the shell to access the sample, and conducting at least one test on the sample to characterize a residual powder material that remains subsequent to the fabrication of the at least one component. In some embodiments, the at least one test incudes at least one of: a first test to determine a chemical composition of the sample, a second test to determine a particle distribution of the sample, a third test to determine a morphology of the sample, a fourth test to determine a flow capability of the sample, a fifth test to determine a moisture content of the sample, and a sixth test to measure a density of the sample. In some embodiments, the method comprises determining, based on an output of the at least one test, a reuse number for the residual powder material, wherein the reuse number indicates a number of reuses of the residual powder material before the residual powder material is discarded or reallocated to an alternative use. In some embodiments, the method comprises determining a difference between an output of the at least one test and a model, updating the model based on the determined difference, and utilizing the updated model to generate a prediction regarding a reuse number of the powder, wherein the reuse number is generated for an alternate build configuration of a second component that is of the same type as the at least one component without requiring experimentation. In some embodiments, the method comprises determining that the at least one component consumes a fraction of a surface area of a build plate, and scaling the core based on the fraction.

### BRIEF DESCRIPTION OF THE DRAWINGS

The present disclosure is illustrated by way of example and not limited in the accompanying figures in which like reference numerals indicate similar elements. The figures are not necessarily drawn to scale unless explicitly indicated otherwise.
FIG. 1 is a side cutaway illustration of a gas turbine engine.
FIGS. 2A and 2B illustrate a top-down view of build plates supporting a fabrication of components and capsules.
FIG. 2C illustrates a component that is fabricated as a plurality of layers on a build plate.
FIG. 3 illustrates a first capsule in accordance with this disclosure.
FIG. 3A illustrates a second capsule in accordance with this disclosure.
FIG. 3B illustrates various profiles of shells and covers in accordance with aspects of this disclosure.
FIG. 4 illustrates a flow chart of a method for fabricating a capsule to characterize residual material.
FIG. 5 illustrates a block diagram of a powder bed fusion environment in accordance with aspects of this disclosure.
FIG. 6 illustrates a system incorporating aspects of a powder bed fusion environment.

### DETAILED DESCRIPTION

It is noted that various connections are set forth between elements in the following description and in the drawings (the contents of which are included in this disclosure by way of reference). It is noted that these connections are general and, unless specified otherwise, may be direct or indirect and that this specification is not intended to be limiting in this respect. A coupling between two or more entities may refer to a direct connection or an indirect connection. An indirect connection may incorporate one or more intervening entities.

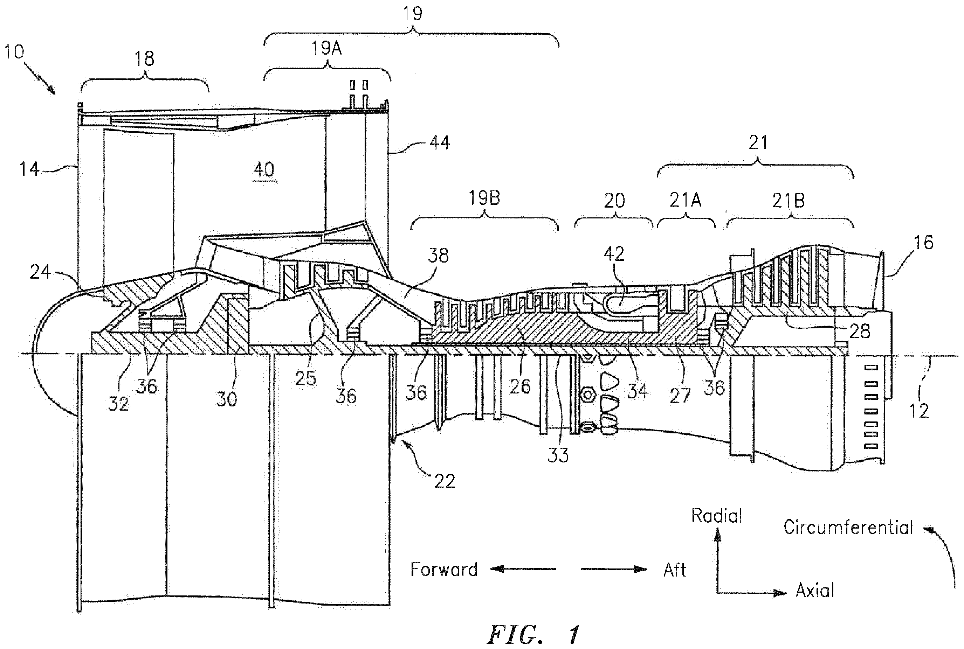

Aspects of the disclosure may be applied in connection with a gas turbine engine. FIG. 1 is a side cutaway illustration of a geared turbine engine 10. This turbine engine 10 extends along an axial centerline 12 between an upstream airflow inlet 14 and a downstream airflow exhaust 16. The turbine engine 10 includes a fan section 18, a compressor section 19, a combustor section 20 and a turbine section 21. The compressor section 19 includes a low pressure compressor (LPC) section 19A and a high pressure compressor (HPC) section 19B. The turbine section 21 includes a high pressure turbine (HPT) section 21A and a low pressure turbine (LPT) section 21B.

The engine sections 18-21 are arranged sequentially along the centerline 12 within an engine housing 22. Each of the engine sections 18-19B, 21A and 21B includes a respective rotor 24-28. Each of these rotors 24-28 includes a plurality of rotor blades arranged circumferentially around and connected to one or more respective rotor disks. The rotor blades, for example, may be formed integral with or mechanically fastened, welded, brazed, adhered and/or otherwise attached to the respective rotor disk(s).

The fan rotor 24 is connected to a gear train 30, for example, through a fan shaft 32. The gear train 30 and the LPC rotor 25 are connected to and driven by the LPT rotor 28 through a low speed shaft 33. The HPC rotor 26 is connected to and driven by the HPT rotor 27 through a high speed shaft 34. The shafts 32-34 are rotatably supported by a plurality of bearings 36 (e.g., rolling element and/or thrust bearings). Each of these bearings 36 is connected to the engine housing 22 by at least one stationary structure such as, for example, an annular support strut.

As one skilled in the art would appreciate, in some embodiments a fan drive gear system (FDGS), which may be incorporated as part of the gear train 30, may be used to separate the rotation of the fan rotor 24 from the rotation of the rotor 25 of the low pressure compressor section 19A and the rotor 28 of the low pressure turbine section 21B. For example, such an FDGS may allow the fan rotor 24 to rotate at a different (e.g., slower) speed relative to the rotors 25 and 28.

During operation, air enters the turbine engine 10 through the airflow inlet 14, and is directed through the fan section 18 and into a core gas path/duct 38 and a bypass gas path/duct 40. The air within the core gas path 38 may be referred to as "core air". The air within the bypass gas path 40 may be referred to as "bypass air". The core air is directed through the engine sections 19-21, and exits the turbine engine 10 through the airflow exhaust 16 to provide forward engine thrust. Within the combustor section 20, fuel is injected into a combustion chamber 42 and mixed with compressed core air. This fuel-core air mixture is ignited to power the turbine engine 10. The bypass air is directed through the bypass gas path 40 and out of the turbine engine 10 through a bypass nozzle 44 to provide additional forward engine thrust. This additional forward engine thrust may account for a majority (e.g., more than 70 percent) of total engine thrust. Alternatively, at least some of the bypass air may be directed out of the turbine engine 10 through a thrust reverser to provide reverse engine thrust.

FIG. 1 represents one possible configuration for an engine 10. Aspects of the disclosure may be applied in connection with other environments, including additional configurations for gas turbine engines. Aspects of the disclosure may be applied in connection with non-geared engines.

As described above, aspects of the disclosure may be used to fabricate/manufacture a component of an engine (e.g., the engine 10 of FIG. 1). Such manufacture may be based on the use of one or more additive manufacturing techniques. In some embodiments, a threshold may be established regarding a reuse of a material (e.g., a powder material) used in the manufacture. The threshold may be based on a representative material collection and characterization technique, potentially in combination with computational and/or analytical modeling.

Via an analysis of a build configuration and machine/process parameters, a capsule may be generated alongside the build configuration that captures residual material in a build chamber that is left-over from one or more prior component builds. The use of a capsule to collect a sample as part of an additive manufacturing technique is known in the art - see, e.g., U.S. patent number 9,778,150.

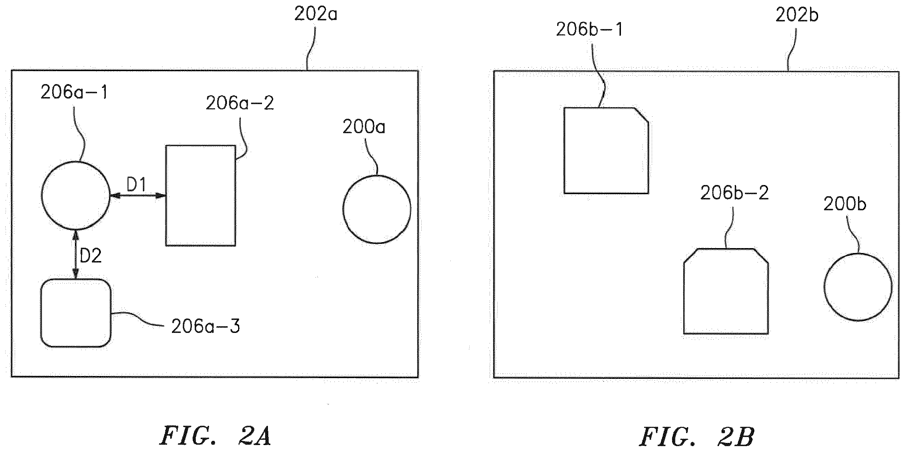

Referring to FIGS. 2A-2B, a build plate 202a and a build plate 202b are shown, respectively. The build plates 202a and 202b may be the same build plate, which is to say that FIGS. 2A and 2B may be representative of two different build instances/procedures/runs. The build plate 202a is shown as supporting a build of various components/workpieces, such as for example components 206a-1, 206a-2, and 206a-3. The build plate 202b is shown as supporting a build of components/workpieces 206b-1 and 206b-2.

The components 206a-1, 206a-2, 206a-3, 206b-1, and 206b-2 may be at least partially differentiated from one another. For example, the component 206a-1 may be a first type of component (e.g., a fuel nozzle), the component 206a-2 may be a second type of component (e.g., a turbine blade), the component 206a-3 may be a third type of component (e.g., a fan blade), the component 206b-1 may be a fourth type of component (e.g., a compressor blade), and the component 206b-2 may be a fifth type of component (e.g., a liner panel of a combustor).

In some embodiments, the components (e.g., the components 206a-1 through 206a-3) on a given build plate (e.g., the build plate 202a) may correspond to multiple instances of a common type of component. Adjacent components may be equidistantly spaced from one another (e.g., distances D1 and D2 in FIG. 2A may be equal to one another) on the build plate, potentially in the form of a grid. By stipulating to these common component and equidistant spacing conditions, the generation of a core of a capsule as described below may be simplified relative to more complex/multi-factored component build conditions.

In addition to supporting a build of different components, the count of components supported by the build plates 202a and 202b may be different from one another. For example, the build plate 202a may be used to build a first number of components and the build plate 202b may be used to build a second number of components, the second number different from (e.g., less than) the first number.

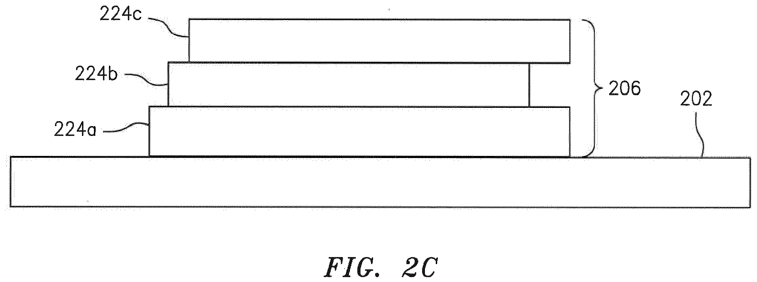

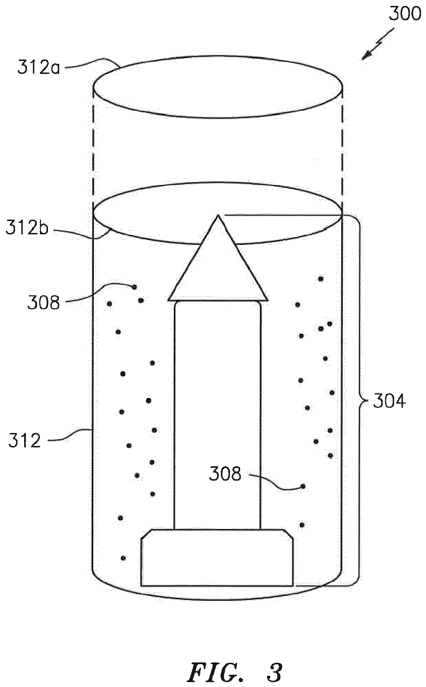

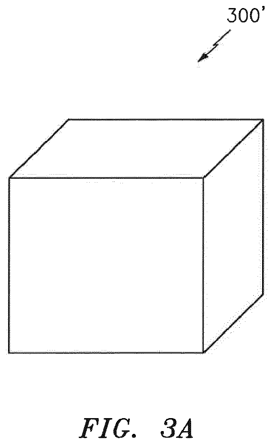

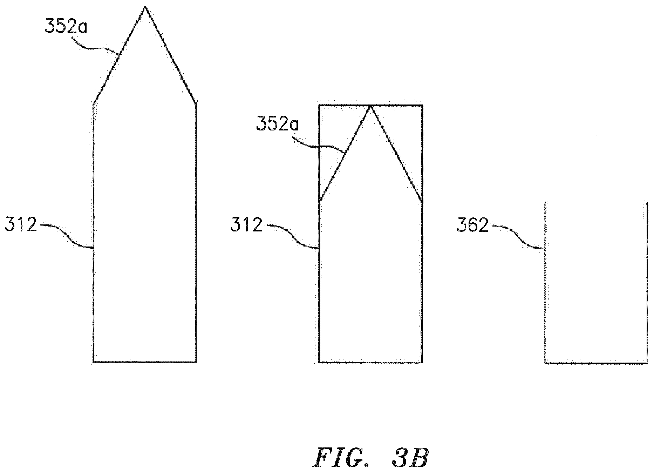

Capsules 200a and 200b may be built alongside components to capture a unique and statistically representative powder sample associated with the build plate 202a or the build plate 202b, respectively. Referring to FIG. 3, a capsule 300 (which may correspond to the capsule 200a or the capsule 200b - see FIGS. 2A-2B) includes the following: (1) a core 304 that is additively manufactured inside of the capsule to be representative of one or more scaled build configuration cross-sections/layers of one or more components (see FIG. 2C - component 206 composed of/built from layers 224a, 224b, and 224c on a build plate 202), (2) sample material (e.g., powder material) 308 that is characteristically representative of the unused powder in a powder bed, (3) a shell 312 that contains the core 304 and the material 308 and (4) a lid/cover that may or may not seal the capsule. The capsule 300 (e.g., the core 304, the material 308, and the shell 312) may be built/generated concurrently with the components (e.g., the components 206b-1 and 206b-2) that are being fabricated on a given build plate (e.g., the build plate 202b) in order to replicate/capture the environmental conditions that are present during the build in connection with the material 308. Thus, much like the components that are being fabricated the capsule 300 may be built/generated in a layer-by-layer manner.

A scaling factor of the core 304 may be based on one or more parameters. For example, the scaling factor may be based on one or more of: a density of material that is used in a build, a density of components that are built relative to, e.g., a surface area of a build plate, an orientation of the components that are built [in terms of, e.g., an orientation of surfaces of the components], an identification/type of the components that are built, laser parameters [e.g. speed and power], and a spacing between the components. As a first-order approximation, if the component(s) that is/are being built on a build plate consume a fraction of a surface area of the build plate, then the scaling factor may be selected to correspond to that same fraction of a surface area or volume of the capsule 300.

While the capsule 300 is shown as being substantially cylindrical in FIG. 3, the capsule 300 may assume one or more other shapes/form-factors. For example, in some embodiments a capsule 300' may be shaped as a square/rectangle/cube (see FIG. 3A). The use of a cube may facilitate the use of a scaling factor in connection with the core 304 as described above; in particular, the geometry of the capsule 300' simplifies the computational complexity associated with the scaling (relative to more complex shapes/form-factors). Other shapes/form-factors that may be used include a rectangular base, a hex base, an octagonal base, etc.

The shell 312 may enclose the core 304 and the material 308 therein. Such an enclosure may help to guard against contamination of the contents of the capsule 300 (e.g., the material 308) and to contain the representative powder sample. In this respect, a cover 312a is shown in a partially exploded/removed position relative to the shell 312, where the cover 312a in combination with the shell 312 may serve to enclose the core 304 and the material 308 therein. While referenced as a separate part, the cover 312a may be manufactured in conjunction with the shell 312 as a unitary piece in some embodiments.

While the capsule 300 is shown in FIG. 3 as being defined by a (substantial) right angle in terms of the interface between, e.g., the shell 312 and the cover 312a, in some embodiments a capsule may include an angle that is less than, e.g., 70 degrees, relative to the vertical reference direction. For example, FIG. 3B illustrates exemplary profiles of capsules that may be used. Such profiles/capsules may facilitate ease in manufacture. For example, in FIG. 3B a cover 352a that is external to the shell 312 is shown, where the cover 352a is characterized by an angle that is less than, e.g., 70 degrees relative to the vertical walls of the shell 312. As manufacturing techniques continue to improve, an allowable build angle from the vertical may tend to increase towards 90 degrees. The cover 352b may be fabricated integral with the shell 312/shell walls, where the angle at the interface between the cover 352b and the vertical walls of the shell 312 may be less than 70 degrees (relative to the vertical direction). As shown in conjunction with the shell 362 (which may correspond to the shell 312), in some embodiments no cover may be included such that (an interior of) the shell 362 maybe open-ended/exposed.

Referring to FIG. 3A, the shell 312 and/or the cover 312a may facilitate transport of the material 308 to a location (e.g., a laboratory) that is remote from a build table for analysis/testing as described below. The shell 312 may include one or more lines 312b of demarcation/weakness. For example, the line 312b may correspond to a perforation that may facilitate breaking open the shell 312 to gain access to the material 308 for analysis/testing. The line 312b may be located at the point that the cover 312a attaches to the (remainder of the) shell 312, such that the cover 312a may be removed to obtain access to the contents of the capsule 300 (e.g., the material 308).

Once the capsule 300 is built/generated, the captured powder 308 may be removed from the capsule, analyzed and results fed into a computational reuse model. The reuse model may be empirically driven. The reuse model may be calibrated by capsule-captured powder and specimen testing. The reuse model may be based on one or more physics-based and/or analytical models.

The reuse model may include results from one or more tests that may be executed to characterize the material 308. For example, a first test may be used to determine a chemical composition of the material 308; a mass spectrometer may be used as part of the first test. A second test may be used to determine a particle size distribution of the material 308; particle scattering may be used as part of the second test. A third test may be used to determine a morphology of the material 308; a microscope (e.g., a scanning electron microscope [SEM]) may be used as part of the third test. A fourth test may be used to determine a flow capability of the material 308; one or more shear/dynamic rheological tests may be used as part of the fourth test. A fifth test may be used to determine a moisture content of the material 308. A sixth test may be used to measure a density (e.g., an apparent density, tap density, skeletal density) of the material 308.

The tests described above are illustrative. In some embodiments, one or more additional tests may be included. In some embodiments, a given test may be optional.

Once the reuse model is calibrated based on material characterization data specific to a given build setup , a permissible reuse number/window on residual material used to fabricate one or more components may be at least partially, and potentially even entirely, model-driven. The reuse model may obtain as inputs one or more of: (1) a build configuration, (2) build theme parameters, (3) material characteristics, or (4) machine characteristics. Based on the inputs, the reuse model may generate and output one or more thresholds/values that correspond to a reuse limitation (for example, a reuse number) for the residual material. The reuse limitation (e.g., reuse number) may provide confidence that the residual material that is to be reused is of a quality for the fabrication of, e.g., aerospace-grade components (e.g., has characteristics that adhere to one or more specifications) and will produce components that are within specified tolerances (for example, the components will include specified material properties). To obtain additional confidence, a capsule may be generated at various reuse numbers/instances and compared to expected model outputs. The comparison may be used to refine the predictive model such that any potential error in using the model may converge towards zero. In this manner, subsequent builds may not incorporate a capsule as the model may accurately predict the reuse number (within an allowable tolerance).

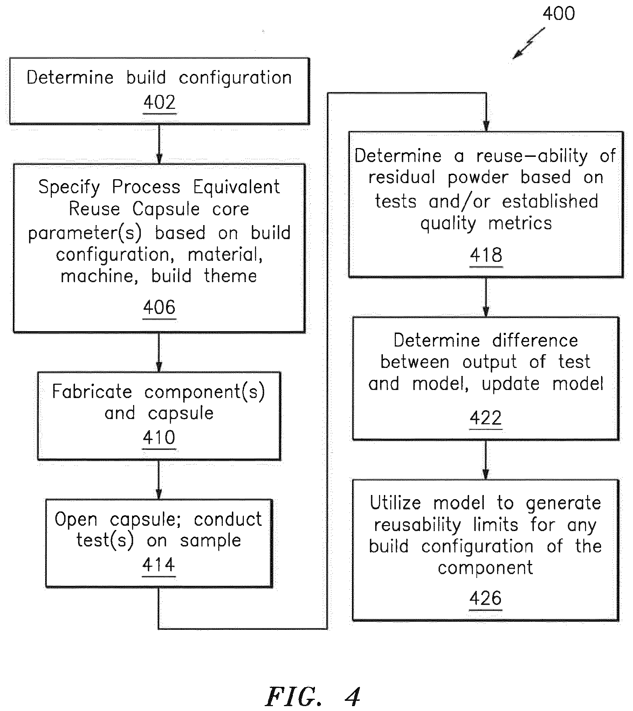

Referring now to FIG. 4, a flow chart of a method 400 is shown. The method 400 may be used to generate a capsule, where a statistically representative sample of material in the capsule may be captured and used to characterize residual material following a fabrication of one or more components using the material.

In block 402, a build configuration may be determined. The build configuration may pertain to a material (e.g., a powder) that is deposited onto a build plate, an identification of (one or more types of) component(s) that are being built on the build plate, and/or an orientation associated with the component(s).

In block 406, one or more parameters of a core may be specified based on the build configuration determined in the block 402. For example, a scaling factor associated with the core may be based on the build configuration of the block 402. The core parameters may also be based on an identification of a material, machine, or build theme. A build theme may include one or more of a build layer height, (laser) scan strategy, machine software, etc.

In block 410, one or more component(s) (which may be specified in the block 402) and a capsule may be fabricated. The fabrication of the block 410 may be based on one or more additive manufacturing techniques and may incorporate a powder material.

In block 414, the capsule of the block 410 may be opened and one or more tests may be conducted to characterize the powder sample contained within the shell. The test(s) may provide one or more outputs that may be used to characterize a residual powder material that remains following the fabrication of the block 410.

In block 418, a statistically-driven reuse limitation (e.g., reuse number) may be determined for the residual powder material based on the test(s) of the block 414 and/or established quality metrics.

In block 422, one or more outputs of the test(s) of the block 414 may be compared to a model (e.g., a reuse model). To the extent that there are any disparities/differences between the output(s) of the test(s) and the model, the model may be updated to support future potential applications of the model (e.g., to support future executions of the block 422, potentially in conjunction with a future build). As part of the block 422, one or more databases may be updated based on the results of the comparison.

In block 426, the updated model may be used to generate a prediction regarding a reuse of the material, such that subsequent material testing may be reduced (e.g., minimized) or even completely avoided for a different build configuration of the same or similar component.

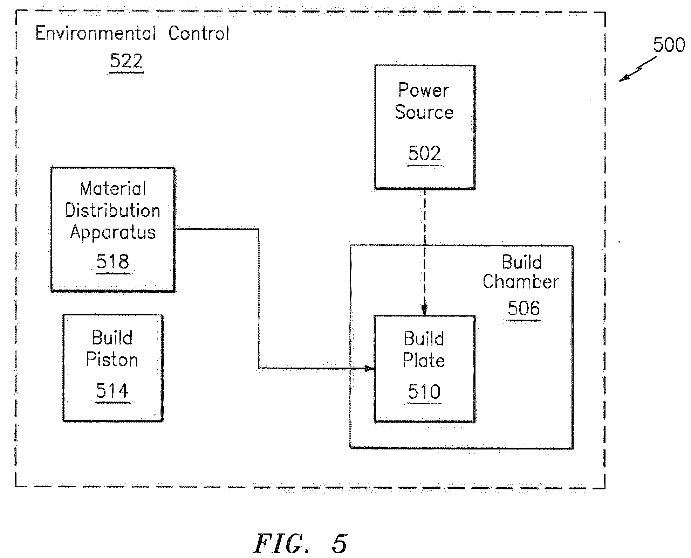

Referring now to FIG. 5, a powder bed fusion environment 500 is shown. The environment 500 may include a power source 502, a build chamber 506, a build plate 510, a build piston 514, a material distribution apparatus 518, and an environmental control 522.

The power source 502 may include, e.g., a laser beam source or an electron beam source. The power source 502 may be used to cause a fusion of material that may be located on the build plate 510. In some embodiments, one or more parameters of the power source 502, such as for example power, frequency, wavelength, or speed, may be controlled (e.g., adjusted/modified) based on whether a component or a capsule is being generated. Such an adjustment may be based on a scaling/scaling factor of the power source 502.

The build chamber 506 may include/define an environment where the build of the component(s) (e.g., the components 206a-1, 206a-2, 206a-3, 206b-1, and/or 206b-2 : see FIGS. 2A-2B) and/or the capsule(s) (e.g., the capsules 200a, 200b, 300 and/or 300' : see FIGS. 2A-2B and 3) occurs.

The build plate 510 (e.g., the build plates 202a and/or 202b : see FIGS. 2A-2B) may provide support to the material that is used in the fabrication of the components and/or the capsules.

The build piston 514, in conjunction with the material distribution apparatus 518, may provide the material to the build plate 510. For example, the build piston 514 may provide material at a surface that is coincident with the material distribution apparatus 518 or the build plate 510. The material distribution apparatus 518 may include a wiper, a blade, etc.

The environmental control 522, which may be applied in conjunction with one or more of the entities (e.g., the build chamber 506) shown in FIG. 5, may be used to provide for atmospheric control during the fabrication of the components and/or the capsules. The environmental control 522 may include/provide one or more inert gases, a vacuum, etc.

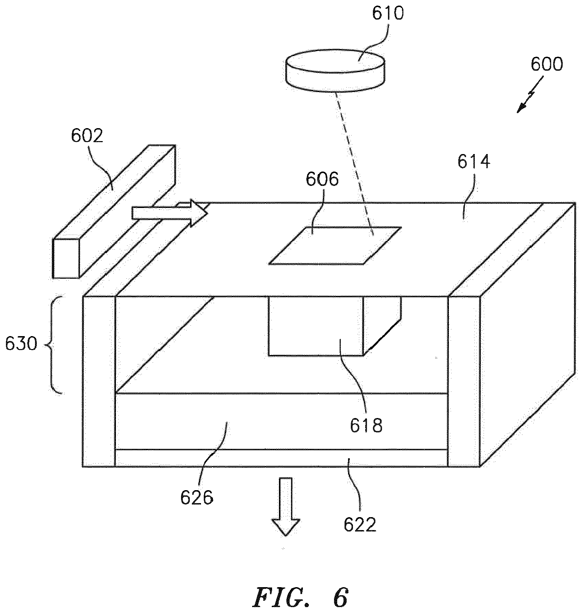

FIG. 6 illustrates a system 600 that may be used in conjunction with one or more powder bed additive manufacturing techniques/systems, such as for example the system 500 of FIG. 5. The system 600 may include a recoater/recoating system 602, a slice/melted powder 606, a power source 610, a powder bed/current layer 614, (previous) layers of a fabricated component 618, a processing table 622, a base/build plate 626, and a volume filled with powder 630.

The recoater 602 may spread powder onto the powder bed 614. The power source 610 may fuse a specified section/portion of the powder bed 614 to form a cross-section of the component (as represented by the slice 606, illustratively shown as being located above the previous layers 618). A build piston (e.g., build piston 514 of FIG. 5) may move, e.g., down (moving the build plate 626 down), and more powder from the volume 630 may be spread by the recoater 602 onto the powder bed 614. The procedure may repeat until the full component is fabricated. The processing table 622 may provide support to, e.g., the build plate 626.

The generation of a capsule as described herein may be used to obtain a sample of material that is (statistically) representative of a residual material that is present following a fabrication/manufacture of one or more components. Representativeness of the powder originates from the unique capsule-core design which is unique to each build setup. In this manner, one or more characteristics of the residual material may be determined based on an analysis/test of the sample. These characteristics may be assessed to determine whether the residual material may be used for a particular purpose, e.g., whether the residual material may be reused in a subsequent component manufacture.

Aspects of the disclosure may be used to generate capsules that capture powder samples that are representative of a material used to manufacture/build one or more components. The capsules contain an additively manufactured core that is designed to mimic the build configuration enabling the capture of a residual powder sample that is representative (in size distribution and properties) of the entire population of unused powder for the given build. Characteristics of the material obtained from the capsule may be used to generate and/or refine a model that may predict reuse windows for one or more build configurations, thus generalizing reuse number "point solutions" to any build configuration of a component. Accordingly, a material-component-property linkage/relationship may be established for qualification of material reuse in additively manufactured components. Aspects of the disclosure may yield a methodology for standardizing material reuse, thereby saving time and cost during component manufacture (e.g., production runs) that reuse material.

Aspects of the disclosure have been described in terms of illustrative embodiments thereof. Numerous other embodiments, modifications, and variations within the scope of the appended claims will occur to persons of ordinary skill in the art from a review of this disclosure. For example, one of ordinary skill in the art will appreciate that the steps described in conjunction with the illustrative figures may be performed in other than the recited order, and that one or more steps illustrated may be optional in accordance with aspects of the disclosure. One or more features described in connection with a first embodiment may be combined with one or more features of one or more additional embodiments.

## Claims

1. A system (600) comprising:
a build plate (202; 202a; 202b; 510; 626);
at least one component (206a-1; 206a-2; 206a-3; 206b-1; 206b-2) that is additively manufactured on the build plate (202...626) from a powder material (308; 630); and
at least one capsule (200a; 200b; 300; 300') that is additively manufactured on the build plate (202...626) from the same powder material (308; 630) that includes
an additively manufactured core (304) that is representative of one or more scaled build configuration layers (224a, 224b, 224c) of the at least one component (206a-1...206b-2) and an arrangement of the layers (224a, 224b, 224c) on the build plate (202... 626);
a sample of unused powder material (308); and
a shell (312; 362) that at least partially encloses the core (304) and the sample (308).

2. The system of claim 1, wherein the at least one component (206a-1...206b-2) includes a plurality of components (206a-1...206b-2), wherein, optionally:
each of the plurality of components (206a-1...206b-2) is an instance of a common type of component (206a-1...206b-2); and/or
components (206a-1...206b-2) that are adjacent to one another on the build plate (202...626) are equidistantly spaced from one another.

3. The system of claim 2, further comprising:
a power source (610) that is applied to the powder material (308; 630),
wherein a geometry of the core (304) is based on a scaling factor derived from at least one of: a density of the powder material (308; 630), a density of the plurality of components (206a-1...206b-2) relative to a surface area of the build plate (202...626), an orientation of the plurality of components (206a-1...206b-2), a type of the components (206a-1...206b-2), and a spacing between the plurality of components (206a-1.. ,206b-2).

4. The system of claim 1, further comprising:
a cover (312a; 352a) that when coupled to the shell (312; 362) completely encloses the core (304) and the sample (308), and wherein the cover (312a; 352a) is removable from the shell (312; 362) to enable access to the sample (308).

5. A capsule (200a...300') comprising:
a core (304) that is representative of one or more scaled build configuration layers (224a, 224b, 224c) of at least one additively manufactured component (206a-1...206b-2);
a sample of unused powder material (308) captured in-situ during fabrication of the at least one additively manufactured component (206a-1...206b-2); and
a shell (312; 362) that at least partially encloses the core (304) and the sample (308),
wherein the sample (308) has properties that are statistically representative of an entire population of the unused powder material (308; 630) on a powder bed (614).

6. The capsule of claim 5 or system of any of claims 1 to 3, wherein the shell (312) completely encloses the core (304) and the sample (308), wherein, optionally, the shell (312; 362) includes a perforation (312b) to enable access to the sample (308).

7. The capsule or system of any preceding claim, wherein the capsule (200a...300') is shaped as at least one of a cylinder (300) or a cube (300').

8. The capsule of any of claims 5 to 7, wherein the core (304) is representative of a plurality of scaled build configuration layers (224a, 224b, 224c) of a plurality of additively manufactured components (206a-1...206b-2), the plurality of additively manufactured components (206a-1...206b-2) including the at least one additively manufactured component (206a-1...206b-2).

9. The capsule of any of claims 5 to 8, wherein the shell (312; 362) is made of at least a portion of the powder material (308; 630).

10. A method comprising:
fabricating at least one component (206a-1...206b-2) from a powder material (308; 630) as a plurality of layers (224a, 224b, 224c) via a powder bed fusion additive manufacturing technique; and
generating a capsule (200a...300') concurrent with the manufacture of the at least one component (206a-1...206b-2), the capsule (200a...300') including a core (304) that is representative of one or more scaled build configuration layers (224a, 224b, 224c) of the at least one component (206a-1...206b-2), a sample of the powder material (308), and a shell (312; 362) that at least partially encloses the sample (308).

11. The method of claim 10, wherein the shell (312; 362) completely encloses the core (304) and the sample (308), the method further comprising:
opening the shell (312; 362) to access the sample (308); and
conducting at least one test on the sample (308) to characterize a residual powder material that remains subsequent to the fabrication of the at least one component (206a-1...206b-2).

12. The method of claim 11, wherein the at least one test incudes at least one of:
a first test to determine a chemical composition of the sample (308);
a second test to determine a particle distribution of the sample (308);
a third test to determine a morphology of the sample (308);
a fourth test to determine a flow capability of the sample (308);
a fifth test to determine a moisture content of the sample (308); and
a sixth test to measure a density of the sample (308).

13. The method of claim 11 or 12, further comprising:
determining, based on an output of the at least one test, a reuse number for the residual powder material (308; 630),
wherein the reuse number indicates a number of reuses of the residual powder material (308; 630) before the residual powder material (308; 630) is discarded or reallocated to an alternative use.

14. The method of any of claims 11 to 13, further comprising:
determining a difference between an output of the at least one test and a model;
updating the model based on the determined difference; and
utilizing the updated model to generate a prediction regarding a reuse number of the powder (308; 630),
wherein the reuse number is generated for an alternate build configuration of a second component that is of the same type as the at least one component (206a-1...206b-2) without requiring experimentation.

15. The method of any of claims 10 to 14, further comprising:
determining that the at least one component (206a-1...206b-2) consumes a fraction of a surface area of a build plate (202...626); and
scaling the core (304) based on the fraction.
